Europäisches Patentamt

European Patent Office

Office européen des brevets

(19)

(11) **EP 0 185 595 B2**

(12) # NOUVEAU FASCICULE DE BREVET EUROPEEN

(45) Date de publication et mention de la
décision concernant l'opposition:
**09.12.1998  Bulletin 1998/50**

(51) Int Cl.⁶: **C07H 1/08**, A23L 1/236

(45) Mention de la délivrance du brevet:
**03.04.1991  Bulletin 1991/14**

(21) Numéro de dépôt: **85402588.9**

(22) Date de dépôt: **20.12.1985**

(54) **Procédé de préparation d'un produit à haute teneur en maltitol et applications de ce produit**

Verfahren zur Herstellung eines Produktes mit einem hohen Gehalt an Maltitol und Anwendung dieses
Erzeugnisses

Process for preparing a product with a high maltitol content, and uses of this product

(84) Etats contractants désignés:
**BE CH DE FR GB IT LI NL SE**

(30) Priorité: **20.12.1984  FR 8419601**

(43) Date de publication de la demande:
**25.06.1986  Bulletin 1986/26**

(73) Titulaire: **Roquette Frères
62136 Lestrem (FR)**

(72) Inventeurs:
• **Devos, Francis
Morbecque F-59190 Hazebrouck (FR)**
• **Gouy, Pierre-Antoine
F-59130 Lambersart (FR)**

(74) Mandataire: **Koch, Gustave et al
Cabinet PLASSERAUD
84, rue d'Amsterdam
75440 Paris Cédex 09 (FR)**

(56) Documents cités:
| | |
|---|---|
| **EP-A- 0 072 080** | **DE-A- 2 034 700** |
| **FR-A- 2 000 580** | **FR-A- 2 000 581** |
| **FR-A- 2 201 042** | **FR-A- 2 275 555** |
| **FR-A- 2 454 830** | **FR-A- 2 499 576** |
| **GB-A- 1 454 697** | **GB-B- 2 097 004** |
| **JP-A- 2 290 465** | **US-A- 3 565 765** |
| **US-A- 3 708 396** | **US-A- 4 246 431** |
| **US-A- 4 346 116** | **US-A- 4 422 881** |

EP 0 185 595 B2

## Description

L'invention a pour objet un nouveau procédé de fabrication d'un produit à haute teneur en maltitol.

Elle vise également le produit obtenu par ce procédé et les applications de ce produit. On rappelle que le maltitol ou ($\alpha$-D-glucopyranosyl)-4-D-sorbitol ou encore (4-O-$\alpha$-D-glucopyranosyl)-D-glucitol constitue le résultat de l'hydrogénation du maltose.

On connait différents procédés permettant d'obtenir des sirops riches en maltose.

Parmi ces procédés, on peut citer :

- celui décrit par HODGE et coll. dans "Cereal Chemistry" n°25, pages 19-30, janvier 1948, et qui comprend une étape de précipitation des dextrines limites par des solutions alcooliques,
- celui décrit par WOLFROM et THOMPSON dans "Methods in carbohydrate chemistry", 1962, pages 334-335, et qui comprend une étape de cristallisation répétée de l'octaacétate de maltose suivie de la cristallisation du maltose,
- celui décrit dans le brevet US 4.294.623 de MEIJI SEIKA accordé le 13.10.81 et qui comprend une étape d'adsorption sur charbon des dextrines,
- celui décrit dans le brevet FR 2.510.581 de HAYASHIBARA déposé le 03.08.82 et qui comprend une étape de chromatographie zur zéolithes ou résines cationiques ou anioniques.
- celui décrit dans le brevet US 4.429.122 de U.O.P. et qui comprend une étape d'ultrafiltration des sirops de maltose, et
- celui qui est décrit dans le brevet FR 2.012.831 de HAYASHIBARA déposé le 27.03.70 et qui comprend l'utilisation combinée de plusieurs enzymes différentes, à savoir une $\alpha$-amylase, une $\beta$-amylase et une isoamylase pullulanase.

Cette dernière technique présente, par rapport aux précédentes, de nombreux avantages.

Elle souffre néanmoins de certains inconvénients, dont notamment :

- celui résidant dans le fait que la liquéfaction doit être effectuée à des valeurs de DE très faibles, une rétrogradation intempestive de l'amylose à ces faibles taux d'hydrolyse gênant les opérations suivantes de saccharification et de purification,
- celui résidant dans le fait que les saccharifications doivent être effectuées à des teneurs en matières sèches très basses, de l'ordre de 20 g/l, pour obtenir une efficacité d'hydrolyse maximum des enzymes.

Le document DE 20 34 700 décrit des édulcorants pour l'alimentation et pour des boissons, ces édulcorants étant constitués par des sirops contenant du maltitol, du maltotriitol et des oligosaccharides hydrogénés supérieurs tels que le maltotétraitol dont les inconvénients sont connus.

Le brevet français N° 2.000.580 décrit un procédé de préparation d'un sirop à teneur élevée en maltitol par hydrogénation d'un sirop à teneur élevée en maltose qui et obtenu par liquéfaction d'un lait d'amidon à faible teneur en matières sèches jusqu'à un dextrose-équivalent inférieur à 2, le produit ainsi obtenu étant saccharifié sous l'action d'enzymes spéciales.

Ce procédé est d'un rendement médiocre, il est coûteux, il donne lieu à des problèmes de contamination bactérienne, il donne lieu à rétrogradation de l'amylose et le sirop obtenu contient des proportions de polyols de DP $\geqq$ 4 qui sont gênantes.

Il s'ensuit qu'aucune de ces techniques ne permet d'obtenir de façon aisée et économique des sirops à haute teneur en maltose propres à fournir par hydrogénation des sirops à haute teneur en maltitol.

De plus, ces techniques ne permettent pas d'obtenir facilement des produits d'une pureté suffisante.

En particulier, ils présentent notamment une teneur en produits de DP $\geqq$ 4 supérieure à 1% en poids par rapport à la matière sèche.

Or, il se trouve que la Société Demanderesse a mis au point un nouveau procédé extrêmement performant et apte à fournir aisément un produit à haute teneur en maltitol.

Ce procédé fournit simultanément un produit riche en maltotriitol.

Ce nouveau procédé permet d'utiliser des sirops de maltose à richesse intermédiaire (contenant de 50 à environ 80% de maltose) obtenus facilement et de façon économique.

Il repose essentiellement sur la découverte du fait que tout sirop de maltose contenant plus de 50% en poids de maltose sur la matière sèche est propre à fournir, après hydrogénation et par fractionnement chromatographique, notamment un sirop riche en maltitol pratiquement exempt de polysaccharides hydrogénés de DP égal ou supérieur à 4 et contenant très peu de sorbitol et de maltotriitol.

Il s'ensuit que le procédé conforme à l'invention pour la préparation simultanée d'un sirop riche en maltitol propre à l'obtention de maltitol cristallisé et d'un sirop riche en maltotriitol comprenant

- la liquéfaction d'un lait d'amidon,
- le traitement du lait d'amidon liquéfié par l'action d'une enzyme saccharifiante en vue de l'obtention d'un sirop de maltose,
- l'hydrogénation catalytique dudit sirop de maltose à l'aide de catalyseurs à base de Ruthenium ou de nickel Raney en vue de l'obtention d'un sirop de maltitol, est caractérisé par le fait
- que l'on soumet un lait d'amidon ayant une teneur en matières sèches de 25 à 45% en poids à une liquéfaction jusqu'à une valeur de dextrose-équivalent égale ou supérieure à 2,
- que l'on soumet le lait d'amidon liquéfié à une saccharification enzymatique jusqu'à obtention d'un sirop de maltose ayant une teneur en maltose de seulement 50 à 80% en poids,
- que l'on hydrogène ledit sirop de maltose par voie catalytique en vue de l'obtention d'un sirop de maltitol, ce sirop contenant également du sorbitol, du maltotriitol et des polyols de degré de polymérisation $\geqq 4$,
- que l'on soumet ledit sirop de maltitol à un fractionnement chromatographique fournissant une fraction riche en sorbitol, une fraction riche en polyols de degré de polymérisation $\geqq 4$, une fraction de sirop riche en maltotriitol et une fraction de sirop riche en maltitol, les conditions opératoires de ladite étape de fractionnement chromatographique ayant été choisies de telle sorte que ladite fraction de sirop riche en maltitol comprend, les pourcentages étant exprimés en poids sur la base de la teneur en matières sèches:

> au moins 87% de maltitol, de préférence de 87 à 97.5% et, encore plus préférentiellement, de 87 à 95.5%,
> moins de 1% de polyols de degré de polymérisation $\geqq 4$, de préférence moins de 0,70% et, plus préférentiellement encore, moins de 0,60%,
> moins de 5% de sorbitol, de préférence moins de 3% et, encore plus préférentiellement, moins de 2%,
> une proportion de maltotriitol comprise entre 2,5 et 13% et,

- que l'on récupère ladite fraction de sirop riche en maltitol.

Subséquemment, on amène par concentration ou par dilution au moins les fractions riches en maltitol à la teneur en matières sèches désirée.

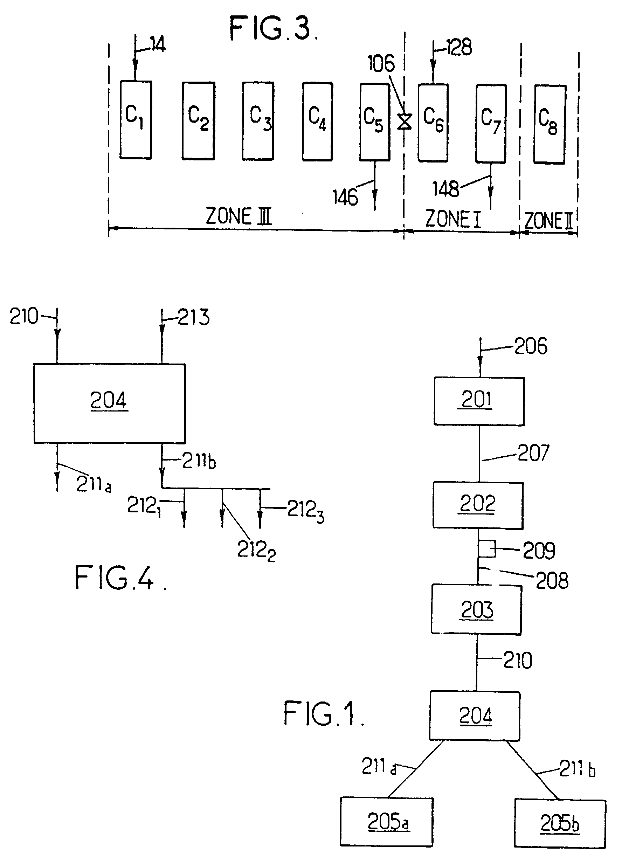

Plus précisément, le procédé objet de l'invention peut être mis en oeuvre à l'aide de l'installation schématiquement représentée à la figure 1 et qui comprend :

- une enceinte 201 à l'intérieur de laquelle on procède à la liquéfaction de l'amidon,
- une enceinte 202 à l'intérieur de laquelle on procède à la saccharification de l'amidon,
- une enceinte 203 à l'intérieur de laquelle on procède à l'hydrogénation catalytique,
- une enceinte 204 de séparation chromatographique,
- une ou plusieurs enceintes 205a, 205b, ..., permettant de concentrer aux teneurs en matières sèches souhaitées en alternance ou chacune en continu les diverses fractions issues de la chromatographie.

L'enceinte 201 est alimentée par une canalisation 206 en lait d'amidon ou de fécule additionné d'acide dans le cas d'une liquéfaction dite acide, ou d'une $\alpha$-amylase dans le cas d'une liquéfaction enzymatique, dans les conditions de teneur en matières sèches, de pH, de taux d'enzyme et de calcium connues de l'homme de l'art pour obtenir un DE (dextrose équivalent) égal ou supérieur à 2.

L'enceinte 202 est alimentée par une canalisation 207 en sirop d'amidon liquéfié sortant de l'enceinte 201 ; avant son entrée dans l'enceinte 202, on ajoute au sirop sortant de l'enceinte 201 , une amylase de malt et éventuellement une enzyme hydrolysant les liaisons $\alpha$ 1-6 ; on procède dans des conditions et de façon connues en elles-mêmes de telle façon qu'on obtienne une richesse en maltose de 50 % au moins à la sortie de l'enceinte 202.

L'enceinte 203 eut alimentée à partir de l'enceinte 202 et par une canalisation 208 en sirop saccharifié, puis filtré et déminéralisé dans un dispositif 209 placé sur la canalisation 208.

On procède dans l'enceinte 203 à l'hydrogénation catalytique du sirop de maltose dans les conditions bien connues de l'homme de l'art, notamment avec des catalyseurs au ruthénium ou au nickel de Raney.

De préférence, l'étape d'hydrogénation est effectuée avec un catalyseur au nickel de Raney, à une pression d'hydrogène supérieure à 20 kg/cm$^2$ et, de préférence, comprise entre 40 et 70 kg/cm$^2$ et à une température d'environ 100 à 150° C. L'hydrogénation est poursuivie jusqu'à ce que la teneur en sucres réducteurs du sirop hydrogéné soit inférieure à 2 %, de préférence inférieure à 1 % et, plus préférentiellement encore, inférieure à 0.5 % (la teneur en sucres réducteurs étant définie en poids d'équivalent dextrose par rapport à la matière sèche).

L'enceinte 204 est alimentée par une canalisation 210 en sirop hydrogéné sortant de l'enceinte 203, ce sirop étant purifié et concentré sur des dispositifs non représentés figure 1.

On procède dans l'enceinte 204 au fractionnement chromatographique.

On achemine par des canalisations 211a, 211b ... les fractions sortant de l'enceinte 204.

L'étape de fractionnement chromatographique peut être effectuée de manière connue en soi, de façon discontinue ou continue (lit mobile simulé), sur des adsorbants du type résines cationiques fortement acides, chargées en ions alcalins ou alcalino-terreux.

Des exemples de tels procédés de séparation chromatographique sont donnés dans les brevets US 3.044.904, US 3.416.961, US 3.692.582, FR 2.391.754, FR 2.099.336. US 2.985.589, US 4.024.331, US 4.226.977, US 4.293.346. US 4.157.267, US 4.182.633, US 4.332.633, US 4.405.445, US 4.412.866 et US 4.422.881.

Suivant un mode de réalisation préféré, l'étape de séparation est effectuée par mise en oeuvre du procédé et de l'appareillage décrits dans le brevet US N° 4.422.881 et son correspondant français N° 79 10563 dont la Société Demanderesse est titulaire.

Quel que soit le procédé de séparation chromatographique retenu, on a recours, de préférence, à titre d'adsorbant, à une résine cationique forte mise sous forme calcium et ayant un taux de divinylbenzène d'environ 4 à 10 %.

L'efficacité du fractionnement chromatographique permet d'exclure de la fraction maltitol, les produits hydrogénés de DP supérieur ou égal à 4 d'une façon quasi totale, même si les sirops soumis au fractionnement en contiennent des quantités notables, par exemple comprises entre 5 et 40 %. On peut donc avoir recours à des hydrolysats d'amidon dont la richesse en maltose n'est que de 50%.

Il s'ensuit que les laits d'amidon peuvent être mis en oeuvre à des teneurs en matières sèches élevées, en tout cas comprises entre 25 et 45 % et voisines de 40 %, comme celles qu'il est habituel de mettre en euvre dans les glucoseries-dextroseries.

Ainsi, les volumes à traiter sont bien moindres que dans les procédés antérieurs, l'énergie nécessaire à l'évaporation de l'eau se trouve fortement réduite, la liquéfaction de l'amidon peut se faire à un DE supérieur à 2, compatible avec une absence de rétrogradation de l'amidon, l'emploi d'enzymes coûteuses comme l'isoamylase ou la pullulanase peut être évité, les hautes pressions osmotiques occasionnées par la concentration élevée des sirops mettant ceux-ci en outre à l'abri de toute contamination microbienne.

Dans le procédé conforme à l'invention, la liquéfaction des laits d'amidon peut être effectuée par voie acide ou enzymatique.

Les conditions de la saccharification enzymatique :

- type d'enzyme ($\beta$-amylase d'origine végétale ou bactérienne, en combinaison ou non avec une enzyme débranchante).
- quantité d'enzyme,
- température d'amylolyse,
- pH d'amylolyse et
- durée d'amylolyse.

sont généralement choisies de façon telle que la teneur en maltose du sirop obtenu soit de 50 à 80 % et, de préférence, de 60 à 80 % sur la matière sèche.

Dans le procédé conforme à l'invention, on choisit les paramètres de l'étape de séparation chromatographique de telle sorte qu'on obtienne, outre un sirop enrichi en maltotriitol et un sirop composé de produits hydrogénés à haut poids moléculaire, un sirop de maltitol présentant une richesse au moins égale à 87 % en poids de maltitol, préférentiellement de 87 à 97,5 % et plus préférentiellement encore, de 87 à 95,5 % de maltitol, contenant moins de 1 % de maltotétraitol et de produits hydrogénés de masse molaire supérieure.

Le choix des paramètres de l'étape de chromatographie, dont notamment :

- le débit d'élution,
- le débit d'alimentation en sirop hydrogéné,
- le débit d'extraction de la fraction riche en maltitol,
- la composition des zones de désorption, d'adsorption et d'enrichissement,

se trouve expliqué et illustré dans les exemples.

De préférence, le susdit sirop de maltitol contient une proportion en sorbitol inférieure à 5 %, de préférence inférieure à 3 % et, encore plus préférentiellement, inférieure à 2 %.

Sa teneur en maltotriitol est généralement comprise entre 2,5 et 13 % en poids.

Le produit obtenu conformément à l'invention peut se présenter sous la forme d'un produit liquide, généralement commercialisé à une matière sèche supérieure à 65 %, ou encore sous la forme d'une poudre sèche, non hygroscopique, obtenue à partir dudit produit liquide, par exemple par solidification et séchage de solutions très concentrées.

Ce produit, sous sa forme physique adaptée, peut être utilisé en tant qu'agent édulcorant ou humectant dans les "produits comestibles", expression par laquelle on entend les produits destinés à l'absorption orale, tels que diverses denrées alimentaires comme les confiseries, les pâtisseries, les crèmes, les boissons et les confitures, ainsi que des produits pharmaceutiques, diététiques, cosmétiques ou hygiéniques, tels que par exemple les élixirs et sirops contre la toux, les tablettes ou comprimés et les pâtes dentifrices, opaques ou transparentes.

Il présente en effet plusieurs avantages qui le destinent tout particulièrement à ces applications.

L'innocuité de son constituant principal, le maltitol, est parfaitement démontrée et reconnue.

Le maltitol est une substance métabolisée au niveau du tube digestif. Il a ainsi été démontré chez l'animal que le complexe maltase-glucoamylase ("New approach to the metabolism of hydrogenated starch hydrolysate". Sous presse. ROSIERS C., VERWAERDE F., DUPAS H., BOUQUELET S., Laboratoire de chimie biologique, Université des Sciences et Techniques de LILLE I et Laboratoire associé au C.N.R.S. n° 217 -Directeur : J. MONTREUIL- 59655 VILLENEUVE D'ASCQ CEDEX, France), présent notamment au niveau du duodénum, hydrolyse la liaison glucose $\alpha$-1,4-sorbitol, libérant ainsi une molécule de sorbitol dont l'absorption est réalisée par voie passive, et une molécule de glucose dont le passage à travers la muqueuse intestinale se fait par transfert actif avec l'intervention d'une adénosine-triphosphatase membranaire sodium et potassium dépendante ("Sugars in Nutrition" SIPPLE H.L., MAKINEN K.N., Acad. Press 1974).

Les études entreprises chez l'homme ont confirmé les résultats obtenus chez l'animal. Le métabolisme du maltitol libère du glucose et du sorbitol, deux molécules dont le devenir métabolique est bien connu ("Digestion of maltitol in Man, Rat and Rabbit" ZUNFT M.J., SCHULZE J., GARTNER H., GRUTTE F.K.; Ann. Nutr. Metab. 27 (1983) p. 470-476).

La digestion de ce produit se traduit par une élévation de la glycémie et de l'insulinémie. Il apparait cependant que l'élévation de la glycémie est plus étalée dans le temps que celle observée après l'absorption d'une charge équivalente de glucose ou de saccharose ("The metabolic fate of hydrogenated glucose syrups" KEARSLEY M.W., BIRCH G.C., LIAN-LOH R.H.P., "Die Stärke" 34 (1982) Nr.8, p. 279-283). Les dosages urinaires et fécaux indiquent qu'une faible quantité de maltitol peut échapper à l'hydrolyse intestinale. Néanmoins, selon différents auteurs, l'utilisation calorique du maltitol se situerait entre 90 et 99 % de la dose ingérée ("Metabolism and caloric utilization of orally administered Maltitol 14C in rat, dog and man", RENNHARD H.H., BIANCHINE J.R.; Journal Agric. Food Chem., Vol. 24, n° 2, 1976, p. 287-291). En conséquence, on peut prévoir que la tolérance digestive du produit obtenu selon l'invention sera liée à celle du glucose et du sorbitol et qu'elle sera donc excellente, tant chez l'enfant que chez l'adulte.

Le pouvoir sucrant du produit obtenu conformément à l'invention est d'autre part élevé, proche de celui du saccharose.

Son pouvoir humectant et ses propriétés de rétention de l'humidité sont très avantageuses dans certains produits de confiserie ainsi que dans les dentifrices et les gels.

Il est en outre stable à la chaleur, ne provoquant pas de réactions de caramélisation ou de brunissement par chauffage en présence de protéines.

De par sa composition très particulière, caractérisée par une très forte teneur en maltitol et surtout par une teneur extrêmement faible en oligosaccharides hydrogénés de DP supérieur ou égal à 4, il présente en outre une cariogénéicité extrêmement faible et une viscosité relativement peu élevée et constante.

Il peut également être utilisé dans des domaines tels que les industries du textile, de la fonderie, de la papeterie, en raison notamment de son pouvoir humectant et plastifiant ; il peut également être utilisé en tant qu'intermédiaire chimique ou de synthèse, applications dans lesquelles sa haute richesse en maltitol et sa très faible teneur en polyols de DP supérieur ou égal à 4 peuvent présenter des avantages déterminants, du point de vue par exemple de la viscosité et de l'homogénéité de masse moléculaire et de propriétés des produits finis obtenus ; c'est ainsi qu'il peut être utilisé dans la fabrication de polyuréthanes, dans la préparation de polyesters ou de polyéthers, dans la préparation d'esters d'acides gras.

De par sa très faible teneur en oligosaccharides hydrogénés de DP supérieur ou égal à 4, le produit obtenu conformément à l'invention se prête très facilement à la fabrication de maltitol cristallisé, grâce à la formation spontanée et en grande quantité de cristaux de maltitol.

Cette aptitude particulière est due à la composition originale du produit selon l'invention.

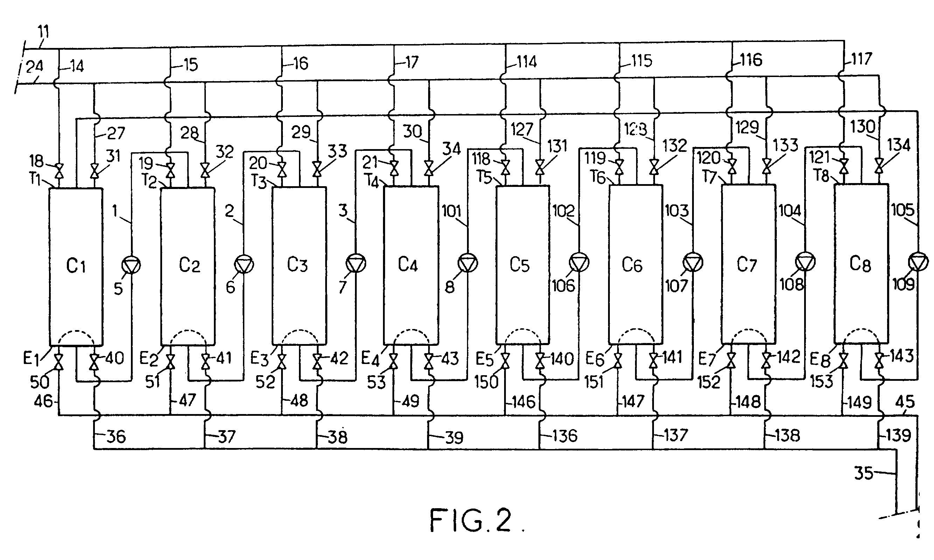

L'invention pourra être encore mieux comprise à l'aide des exemples qui suivent et des figures 2 à 4 qui s'y rapportent, lesdits exemples étant relatifs à des modes de réalisation avantageux de l'invention mais n'en limitant nullement la portée.

Les exemples se divisent en deux groupes.

Le premier groupe englobe les deux premiers exemples ; ceux-ci sont relatifs au nouveau procédé de fabrication du produit riche en maltitol, conformément à l'invention.

Le deuxième groupe englobe les exemples 3 à 6 ; ceux-ci sont relatifs à des applications du produit obtenu conformément à l'invention.

EXEMPLE 1 - PROCEDE DE PREPARATION SELON L'INVENTION D'UN SIROP A HAUTE TENEUR EN MALTITOL A PARTIR D'UN LAIT D AMIDON DE MAIS.

a) Préparation d'un sirop riche en maltose

On a soumis un lait d'amidon de mais à 37 % de matières sèches à une liquéfaction enzymatique classique à l'aide d'$\alpha$-amylase thermorésistante.

On a ajouté ainsi 0,6 ‰ de l'enzyme thermorésistante de la Société NOVO connue sous la dénomination "THERMAMYL" et on a maintenu l'ensemble à 107°C pendant 6 minutes. On a ainsi obtenu une solution à 37 % de matières sèches présentant un DE de 7,5.

On a ajouté ensuite conjointement de la $\beta$-amylase de malt (à savoir 0,5 ‰ de celle connue sous la dénomination "MEZYME" de la Société FINNSUGAR) et de la pullulanase (2 ‰ de celle connue sous la dénomination "PULLUZYME 750L" de A.B.M.), le pH étant de 5,7 et la température de 57° C.

Après avoir maintenu pendant 48 heures l'action conjuguée des deux enzymes, on a obtenu un sirop dont la composition était comme suit:

- dextrose          1,8 % en poids
- maltose          74,9 % en poids
- maltotriose          15,9 % en poids
- Produits de DP 4 à DP 10          4,8 % en poids
- Produits de DP > 10          2,6 % en poids.

b) Préparation d'un sirop riche en maltitol

Après purification sur charbon actif et déminéralisation, on a procédé à l'hydrogénation catalytique du sirop de maltose (teneur en matières sèches de 45 % en poids) dans des conditions classiques, à une température de 125° C et sous une pression d'hydrogène de 55 kg/cm$^2$.

Comme catalyseur, on a utilisé du nickel de Raney.

Le sirop hydrogéné avait la composition suivante après purification sur résines échangeuses d'ions :

- sorbitol          3,2 % en poids
- maltitol          73,1 % en poids
- maltotriitol          14,8 % en poids
- produits de DP 4 à DP 10          5,9 % en poids
- polysaccharides hydrogénés de DP > 10          3,0 % en poids.

c) Etape de fractionnement chromatographique

Le sirop riche en maltitol obtenu à l'étape précédente est concentré à 52,5 % de matières sèches.

Il est ensuite acheminé sur une installation de séparation chromatographique continue dont les détails de la construction et du fonctionnement sont ceux décrits dans le brevet US N° 4.422.881 et dans le brevet correspondant français N° 79 10563, ces détails n'étant repris ici que dans la mesure où la compréhension du texte l'exige.

Cette installation comprend, comme montré à la figure 2 du brevet américain (reprise ici en tant que figure 2, pour l'explicitation détaillée de laquelle on se reportera au brevet américain), huit colonnes ou étages $C_1$ à $C_8$ de 200 titres chacune, garnies d'adsorbant du type résines cationiques fortes sous forme calcium et de fine granulométrie (0,2 à 0,4 millimètres).

De par le calage des électrovannes, on établit une zone 1 de désorption de deux étages, une zone II d'adsorption d'un étage et une zone III d'enrichissement et de séparation des dextrines limites hydrogénées et du maltotriitol, de cinq étages.

Cette disposition se trouve illustrée par la figure 3 qui est une représentation schématique de l'installation selon la figure 2 et sur laquelle on n'a fait figurer que :

- les colonnes $C_1$ à $C_8$,
- le dispositif de fermeture, en l'occurrence l'électrovanne 106,
- les canalisations d'alimentation en sirop de maltitol à fractionner et en eau, montrées respectivement en 14 et 128 et
- les canalisations d'extraction de sirop enrichi en maltitol, d'une part, et d'extractions successives de sorbitol, de dextrines limites et de maltotriitol, d'autre part, montrées respectivement en 148 et 146.

Le dispositif de fermeture 106 maintient dans la configuration adoptée, une étanchéité totale entre, d'une part, la zone III, qui est une zone d'enrichissement à l'extrémité de laquelle sont donc récupérés successivement le reliquat de sorbitol fortement adsorbé, les dextrines limites hydrogénées, puis la fraction enrichie en maltotriitol et, d'autre part, la zone I de désorption du maltitol, zone en tête de laquelle est introduite l'eau de désorption.

Ce dispositif de fermeture assure le sens du passage de la phase liquide sur l'adsorbant sélectif et évite surtout la contamination du maltitol enrichi par des traces de dextrines limites hydrogénées à haut poids moléculaire, dont la vitesse de migration au sein de la résine est largement supérieure à celle du maltitol.

Une minuterie ajustée à 23'30" assure pour les débits indiqués ci-après une alimentation en eau suffisante pour effectuer la désorption de la totalité du maltitol au premier étage de la zone I de désorption, et une alimentation en un volume d'hydrolysat d'amidon hydrogéné riche en maltitol compatible avec le volume d'adsorbant et sa capacité d'adsorption, de façon à obtenir un taux d'extraction de maltitol au moins égal à 90 % du maltitol présent dans l'hydrolysat hydrogéné et cela à une richesse au moins égale à 87 % en maltitol vrai. Le sirop obtenu a une teneur inférieure à 0,5 % de produits de DP supérieur ou égal à 4.

Les susdits taux d'extraction et puretés sont maintenus constants en ajustant le débit de la pompe d'extraction non montrée du maltitol adsorbé. La sortie des fractions "dextrines limites hydrogénées" et" maltotriitol enrichi" s'effectue à pression atmosphérique et son débit constant résulte de la différence entre les débits d'alimentation et le débit d'extraction.

L'hydrolysat d'amidon hydrogéné riche en maltitol qui est introduit dans l'installation en tête de la zone d'enrichissement III, présente, comme indiqué plus haut, une teneur en matières sèches de 52,3 %. La température à l'intérieur des colonnes de séparation est maintenue à environ 90°C.

La figure 4 montre schématiquement en 204 l'installation de la figure 2 (les mêmes références désignant les mêmes éléments pour les parties communes que dans la figure 1). L'installation de chromatographie comporte une canalisation 211b par laquelle sont éliminés les excédents d'eau contenant une fraction importante de sorbitol et la fraction dextrines limites hydrogénées à poids moléculaire supérieur ou égal à DP 4 ; ces extraits sont à faible teneur en matières sèches et sortent par les canalisations $212_1$ et $212_2$.

L'alimentation en eau s'effectue par une canalisa tion 213.

Les flèches portées sur les canalisations indiquent le sens de la circulation.

L'ensemble fonctionne comme suit :

- l'hydrolysat d'amidon hydrogéné devant être soumis au fractionnement chromatographique est acheminé par la canalisation 210 à un débit de 96,9 litres/heure et présente une teneur en matières sèches de 52,3 %,
- l'eau est amenée par la canalisation 213, avec un débit de 393 litres/heure,
- le maltitol enrichi exempt de dextrines limites hydrogénées est récupéré par la canalisation 211a avec un débit de 152 litres/heure, sa teneur moyenne en matières sèches étant de 28,6 %,
- la quantité totale de liquides extraits par ailleurs de l'installation l'est avec un débit total de 337,9 litres/heure, se composant successivement :

  . d'une fraction d'excédent d'eau, extraite par la canalisation $212_1$, contenant, à faible concentration et haute pureté, du sorbitol et d'une fraction à faible concentration et à richesse élevée en dextrines limites hydrogénées à DP supérieur ou égal à 4 extraite par la canalisation $212_2$, l'ensemble représentant un équivalent de 266 litres/heure, la teneur en matières sèches étant de 2,4 % ; ces fractions correspondent aux 18,5 premières minutes du cycle,

  . d'une fraction de maltotriitol enrichie, d'un équivalent de 71,9 litres/heure acheminée par une canalisation $212_3$ vers une installation de purification non montrée, la teneur en matières sèches de cette fraction étant de 10 % : cette fraction correspond aux cinq dernières minutes du cycle.

Les analyses des effluents maltitol enrichi d'une part, de sorbitol, de dextrines limites hydrogénées et de maltotriitol enrichi d'autre part sont, à titre indicatif, données respectivement dans le tableau I pour le maltitol, dans le tableau II pour les autres.

TABLEAU I

| Effluent MALTITOL ENRICHI | | | | | | |
|---|---|---|---|---|---|---|
| Temps | Matière sèche en % | Constituants de DP>4 | DP 4 | DP 3 | Maltitol | Hexitols |
| 0 | 48 | 0 | 0,7 | 12,6 | 86,4 | 0,3 |
| 2 | 47 | 0 | 0,4 | 10,3 | 89,0 | 0,3 |
| 4 | 46 | 0 | 0,3 | 7,2 | 92,1 | 0,4 |

7

TABLEAU I   (suite)

| Temps | Matière sèche en % | Constituants de DP>4 | DP 4 | DP 3 | Maltitol | Hexitols |
|---|---|---|---|---|---|---|
| Effluent MALTITOL ENRICHI | | | | | | |
| 6 | 44,5 | 0 | 0,1 | 5,9 | 93,6 | 0,4 |
| 8 | 44 | 0 | 0 | 4,5 | 94,5 | 1,0 |
| 10 | 39 | 0 | 0 | 3,7 | 95,2 | 1,1 |
| 12 | 35 | 0 | 0 | 3,4 | 95,4 | 1,2 |
| 14 | 30 | 0 | 0 | 3,2 | 95,2 | 1.6 |
| 16 | 24 | 0 | 0 | 3,0 | 95,2 | 1,8 |
| 18 | 19 | 0 | 0 | 3,0 | 95,0 | 2,0 |
| 20 | 14 | 0 | 0 | 2,7 | 94,8 | 2,5 |
| 22 | 9,5 | 0 | 0 | 2,7 | 94,3 | 3,0 |
| 23'30" | - | 0 | 0 | | - | |
| Moyenne | | 0 | 0,2 | 5,9 | 93,3 | 0,6 |

De l'examen du tableau I, il apparaît que le maltitol a été extrait pendant 23'30", à une pureté moyenne de 93,3 % en maltitol et à une teneur en matières sèches moyenne de 28,5 %.

La teneur en hexitol vrai était de 0,6 %, celle des produits de DP 4 hydrogénés de 0,2.

Toutes les dextrines limites hydrogénées supérieures ont été éliminées de cette fraction grâce à cette technologie de fractionnement.

TABLEAU II

| Temps | Matière sèche en % | Constituants de DP>5 | DP 5 | DP 4 | DP 3 | DP 2 | Hexitols |
|---|---|---|---|---|---|---|---|
| Effluent MALTOTRIITOL ENRICHI | | | | | | | |
| 0 | 2,0 | 0 | 0 | 0 | 1,5 | 9,2 | 89,3 |
| 2 | 1,8 | 0 | 0 | 0 | 1,3 | 7,9 | 90,8 |
| 4 | 1,2 | 0 | 0 | 0 | 1,0 | 7,0 | 92,0 |
| 6 | 1,0 | 0 | 0 | 0 | - - | 5,0 | 95 |
| 8 | 0,8 | | | | | | |
| 10 | 0.5 | | | | | | |
| 12 | 0,8 | traces | 0 | 0 | 2,8 | 2,1 | 95,0 |
| 14 | 1,0 | 66,1 | 15,8 | 0 | 1,7 | 4,9 | 11,5 |
| 16 | 2.0 | 46,6 | 21.0 | 11,3 | 17,5 | 1,3 | 2,3 |
| 18 | 4 | 28,0 | 15,7 | 12,6 | 42,3 | 0,7 | 0,7 |
| 20 | 7 | 20,3 | 12,7 | 11,5 | 53,5 | 1,4 | 0,6 |
| 22 | 11 | 16,2 | 10,9 | 10,3 | 59,6 | 2,6 | 0,4 |
| 23'30" | 13 | 16,0 | 10,0 | 10,0 | 59,5 | 3,5 | 1,0 |

L'analyse détaillée des résultats réunis dans ce tableau II montre que :

- pendant les 12 premières minutes, on a éliminé le sorbitol fortement adsorbé dont la richesse moyenne a été supérieure à 85 % et, pour certaines fractions, à 90% ; ceci explique la faible teneur en sorbitol vrai de la fraction maltitol précédemment analysée ;
- puis de la minute 12 à l'instant 18'30", la fraction extraite contenait la majeure partie des dextrines limites hydrogénées supérieures, de DP supérieur ou égal à 4 avec une faible teneur en produits de DP 2 et de DP 3 hydrogénés,
- puis de l'instant 18'30" à l'instant 22'30", on a éliminé la fraction maltotriitol enrichie, à une teneur moyenne de 57,3 %, soit un équivalent de 71 litres/heure à 11 % de matières sèches.

L'installation de fractionnement chromatographique a été alimentée comme montré au tableau III.

TABLEAU III

|  | Hydrolysat hydrogéné riche en maltitol | Eau | Total |
|---|---|---|---|
| Débit | 96,9 l/h | 393 l/h | 89,9 l/h |
| Densité | 1,229 kg/l |  |  |
| Matières sèches | 52,3 % |  |  |
| Débit massique | 62,3 kg/h |  | 62,3 kg/h |
| Richesse en maltitol | 73,1 % |  |  |
| Débit massique en maltitol | 45,54 kg/h |  | 45,54 kg/h |

On a réuni dans le tableau IV les quantités et caractéristiques des effluents retirés de l'installation.

TABLEAU IV

|  | Fraction maltitol | Fraction sorbitol et fraction dextrines réunies | Fraction maltotriitol | Total |
|---|---|---|---|---|
| Débit | 152 l/h | 266 l/h | 71,9 l/h | 489,9 l/h |
| Densité | 1,12 kg/l | 1,00 kg/l | 1,04 kg/l |  |
| Matières sèches | 28,5 % | 2,4 % | 10,0 % |  |
| Débit massique | 48,5 kg/h | 6,4 kg/h | 7,4 kg/h |  |
| Richesse en maltitol | 93,3 % | 7 % | 2 % |  |
| Débit massique en maltitol | 45,25 kg/h | 0,168 kg/h | 0,128 kg/h | 45,54kg/h |

Ces résultats correspondent à un taux d'extraction maltitol de :

$$\frac{45,25}{45,54} = 99\ \%$$

et à un rendement de l'extraction du sirop de maltitol de

$$\frac{48,5}{62,3} = 77,8\ \%.$$

Cette installation de fractionnement chromatographique a ainsi fonctionné pendant quatorze jours. Les tableaux V et VI ci-dessous donnent la composition des extraits en fonction du temps exprimé en jours.

TABLEAU V

| (MALTITOL ENRICHI) | | | | | | | |
|---|---|---|---|---|---|---|---|
| Jours | 2 | 4 | 6 | 8 | 10 | 12 | 14 |
| DP ≥ 4 | 0,2 | 0,2 | 0,3 | 0,1 | 0,4 | 0,6 | 0,1 |
| DP 3 | 5,6 | 6,9 | 5,0 | 5,0 | 5,0 | 5,2 | 4,9 |
| Haltitol | 93,8 | 92,7 | 94,0 | 93,5 | 93,2 | 93,2 | 94,0 |
| Sorbitol | 0,4 | 0,2 | 0,7 | 1,4 | 1,4 | 1,0 | 1,0 |

TABLEAU VI

| (MALTOTRIITOL ENRICHI) | | | | | | | |
|---|---|---|---|---|---|---|---|
| Jours | 2 | 4 | 6 | 8 | 10 | 12 | 14 |
| DP ≥ 4 | 40,5 | 44,8 | 44,6 | - | 36,1 | _ | 37,4 |
| DP 3 | 57 | 51,9 | 53,4 | - | 59,5 | - | 59,6 |
| Maltitol | 2,1 | 2,9 | 1,4 | - | 3,4 | - | 2,6 |
| Sorbitol | 0,4 | 0,4 | 0,6 | - | 1,0 | - | 0.4 |

EXEMPLE 2 - PROCEDE DE PREPARATION SELON L'INVENTION D'UN PRODUIT A HAUTE TENEUR EN MALTITOL A PARTIR D'UN AMIDON DE BLE.

Un lait d'amidon de froment d une teneur en matières sèches de 37 %, a été liquéfié à un pH de 6,3, à une température de 108°C et avec une addition de 0,3 ‰ d'enzyme liquéfiante du type de celle connue sous la marque "THERMAMYL" de la Société NOVO. A la sortie de l'installation de liquéfaction, on a procédé à un choc thermique de 10 secondes à 130° C. Le DE en sortie de liquéfaction était inférieur à 5,0.

On a ajusté le pH à la sortie de l'installation à une valeur de 5.5 et on a ajouté 0.55 ‰ de β-amylase de malt commercialisée sous la dénomination "SPEZYME BBA 1500" par la Société FINNSUGAR. La saccharification a été effectuée à ce pH pendant 48 heures à 57° C.

En fin de saccharification, l'analyse par chromatographie liquide montrait la présence de :

| | |
|---|---|
| Dextrose | 2,3 % en poids |
| Maltose | 61,3 % en poids |
| Trisaccharides | 7.5 % en poids |
| Produits de DP 4 à DP 10 | 6,2 % en poids |
| Produit de DP > 10 | 22,7 % en poids. |

Après hydrogénation effectuée comme décrit dans l'exemple 1, suivie d'une purification et d'une concentration, on a obtenu un sirop riche en maltitol dont la composition était la suivante :

| | |
|---|---|
| Sorbitol | 3,3 % en poids |
| Maltitol | 60,4 % en poids |
| Trisaccharides | 10,2 % en poids |
| Produits de DP 4 à DP 10 | 7,0 % en poids |
| Produit de DP > 10 | 20,1 % en poids. |

On a procédé au fractionnement de cet hydrolysat riche en maltose hydrogéné dans l'installation chromatographique décrite dans l'exemple 1.

On a alimenté l'installation de la manière qui résuite du tableau VII.

TABLEAU VII

| | Sirop maltitol | Eau | Total |
|---|---|---|---|
| Débit | 90 l/h | 430 l/h | 520 l/h |
| Densité | 1,25 kg/l | | |
| Matières sèches | 51,5 % | | |
| Débit massique | 56,3 kg/h | | 56,9 kg/h |
| Richesse en maltitol | 60,4 % | | |
| Débit massique en maltitol | 34 kg/h | | 34 kg/h |

Les effluents extraits de l'installation sont identifiés dans le tableau VIII.

TABLEAU VIII

| | Maltitol enrichi | Sorbitol puis dextrines limites hydrogénées | Maltotriitol | Total |
|---|---|---|---|---|
| Débit | 145 l/h | 305 l/h | 70 l/h | 520 l/h |
| Densité | 1,11 kg/l | 1,02 Kg/l | 1,03 kg/l | |
| Matières sèches | 23 % | 4,5 % | 8,2 % | |
| Débit massique | 37 kg/h | 14,0 kg/h | 5,9 kg/h | 56,9 kg/h |
| Richesse en maltitol | 90,5 % | 2 % | 3,9 % | |
| Débit massique en maltitol | 33,5 kg/h | 0,28 kg/h | 0,23 kg/h | 34,1 kg/h |

Ce résultat correspond à un taux d'extraction massique de :

$$\frac{37}{56,3} = 67\ \%$$

de sirop de maltitol enrichi à 90,5 % et de

$$\frac{33,5}{34} = 98,5\ \%$$

d'extraction du maltitol

L'analyse de la fraction maltitol enrichi donne les résultats suivants :

- Sorbitol     1,3 % en poids
- Maltitol     90,5 % en poids
- DP 3     7,8 % en poids
- DP ≥ 4     0,4 % en poids.

L'analyse de la fraction sirop de maltotriitol enrichi donne les résultats suivants :

- Sorbitol     0,4 % en poids
- Maltitol     3,9 % en poids
- Maltotriitol     51 % en poids
- Maltotétraitol     10,6 % en poids
- produits de DP égal à 5     9,5 % en poids
- Produits de DP > 5     24,6 % en poids.

On peut constater, à partir de ces différentes mesures qu'il a été possible, par ce procédé de fractionnement à partir d'un sirop standard obtenu par saccharifica tion à la β-amylase seule et aux concentrations coutumières élevées, d'extraire à 98,5 % le maltitol à une richesse de 90,5 %.

Ce sirop est lui aussi caractérisé par l'absence quasi totale de dextrines hydrogénées.

On a pu extraire conjointement un sirop enrichi en maltotriitol à une richesse de 51 %.

Dans tous les cas (produits issus des exemples 1 et 2), les effluents très fortement enrichis en maltitol ont été concentrés aux teneurs en matières sèches les plus adaptées aux différentes applications ; ces teneurs en matières sèches sont en général de 65 à 90 %.

Les utilisations découlant des propriétés avantageuses du produit obtenu selon le procédé conforme à l'invention seront mieux comprises à l'aide des exemples suivants relatifs à des modes de réalisation avantageux, mais ne prétendant pas en limiter la portée.

EXEMPLE 3 - APPLICATION DU PRODUIT OBTENU CONFORMEMENT A L'INVENTION A LA FABRICATION DE MALTITOL CRISTALLISE ANHYDRE.

On a recours à un sirop obtenu conformément à l'invention caractérisé par la composition suivante (les pourcentages sont exprimés sur la base sèche) :

| | |
|---|---|
| Sorbitol | 1,1 % en poids |
| Maltitol | 92,4 % en poids |
| Maltotriitol | 6,1 % en poids |
| Produits de DP ≥ 4 | 0,4 % en poids. |

On concentre sous pression réduite jusqu'à une teneur de 90 % de matières sèches à une température de 80° C ce sirop de maltitol.

On recueille cette masse réduite dans un cristallisoir muni d'une double enveloppe. Après quatre heures de maintien à 75° C, on voit apparaitre un début de cristallisation très régulier (nucléation spontanée).

On procède ensuite au refroidissement du cristallisoir à une vitesse de 1° C/heure de 75°C à 25°C en 50 heures.

La masse cristallisée est essorée sur une essoreuse centrifuge.

L'essorage des eaux-mères est très facile, le clairçage (lavage) des cristaux est effectué à l'eau distillée froide.

On recueille après cette opération les cristaux que l'on sèche sur un séchoir à lit fluidisé jusqu'à une teneur en eau inférieure à 0,5 %.

Le maltitol cristallisé a été obtenu avec un rendement de 63 % (par rapport à la matière sèche soumise à la cristallisation) et présente une pureté chimique de 99,2 % par chromatographie liquide haute pression.

Son pouvoir rotatoire (solution aqueuse à 10 %) à 20° C, raie D du sodium est de + 106° C.

Sa teneur en sucres réducteurs est inférieure à 0.01 %.

Sa température de fusion au pic, relevée sur appareil d'analyse thermique différentielle D.S.C. SETARAM 111 avec 50 mg de substance, vitesse de chauffage de 2° C/mn, est de 150,7 ° C.

Il est non hygroscopique et se présente comme une poudre s'écoulant librement.

EXEMPLE 4 - APPLICATION DU PRODUIT OBTENU CONFORMEMENT A L'INVENTION A LA FABRICATION DE SUCRE CUIT

On a recours à un sirop obtenu conformément à l'invention caractérisé par la composition suivante (les pourcentages sont exprimés sur la base sèche) :

| | |
|---|---|
| Sorbitol | 3,4 % en poids |
| Maltitol | 89,2 % en poids |
| Maltotriitol | 6,9 % en poids |
| Produits de DP $\geq$ 4 | 0,5 % en poids. |

Une quantité de 350 g de ce sirop, à 75 % de matières sèches, est chauffée dans un récipient en cuivre sur plaque électrique jusqu'à la température de 180° C avec agitation fréquente.

On laisse refroidir la masse de sirop cuit jusqu'à une température de 130-140° C.

On incorpore soigneusement l'acide, l'arôme et le colorant habituels avant de couler la masse cuite sur un marbre froid.

L'acide utilisé est l'acide citrique.

L'arôme est celui de citron.

La masse refroidie vers 60-80 ° C est découpée en bonbons de 15 x 15 mm.

Les bonbons sont emballés à l'aide de papier étanche.

Ils sont brillants et présentent une saveur sucrée élevée ainsi qu'un goût sucré identique à celui du saccharose.

L'humidité finale des sucres cuits est voisine de 1 %.

La composition en matières sèches du produit fini est la suivante :

| | |
|---|---|
| Sirop de maltitol | 97,8 % en poids |
| Acide citrique | 1 % en poids |
| Arôme citron | 0,2 % en poids |
| Colorant | quantité suffisante |
| Eau | 1 % en poids. |

EXEMPLE 5 - APPLICATION DU PRODUIT OBTENU CONFORMEMENT A L'INVENTION A LA FABRICATION D'UNE BOISSON.

On a recours à un sirop obtenu conformément à l'invention caractérisé par la composition suivante (les pourcentages sont exprimés sur base sèche) :

| | |
|---|---|
| Sorbitol | 2,9 % en poids |
| Maltitol | 91,6 % en poids |
| Maltotriitol | 5,3 % en poids |
| Produits de DP $\geq$ 4 | 0,2 % en poids. |

La composition prévue pour la boisson est la suivante :

- Sirop de maltitol à 75 % de matières sèches      170 g

- Acide citrique     2,6 g
- Pâte aromatique citron     1,4 g
- Eau     quantité supplémentaire pour faire 1 litre.

Pour préparer cette boisson, on mélange le sirop de maltitol à 300 ml d'eau. On ajoute à ce mélange l'arôme et l'acide puis le reste de l'eau.

La boisson est ensuite gazéifiée par injection directe d'anhydride carbonique dans le récipient.

La boisson terminée présente un indice de réfraction de 1.3525. Son niveau sucré est équivalent à celui d une boisson au saccharose, l'impression laissée en bouche est, de plus. très agréable et l'aromatisation est plus perceptible et plus durable que celle de la même boisson préparée avec 100 g/litre de saccharose.

EXEMPLE 6 - APPLICATION DU PRODUIT OBTENU CONFORMEMENT A L'INVENTION A LA FABRICATION DE CHEWING-GUM .

On a recours à un produit obtenu conformément à l'invention caractérisé par la composition suivante (les pourcentages sont exprimés sur base sèche) :

| Sorbitol | 0,7 % en poids |
|---|---|
| Maltitol | 94,8 % en poids |
| Maltotriitol | 4,4 % en poids |
| Produits de DP $\geq$ 4 | 0,1 % en poids. |

Le sirop est concentré à 91 % de matières sèches puis est coulé dans des bassins en polyéthylène.

Après 24 heures de refroidissement à 20° C, les pains blancs de 3 centimètres d'épaisseur sont démoulés et brisés. Les fragments obtenus sont rapés et la poudre est séchée dans un séchoir à lit fluidisé. Elle est alors tamisée pour fournir une poudre s'écoulant librement et répondant à l'analyse granulométrique suivante :

- 0 % de refus sur tamis de 315 microns
- 30 % de particules de dimensions comprises entre 315 et 250 microns
- 35 % de particules de dimensions comprises entre 250 et 160 microns
- 25 % de particules de dimensions comprises entre 160 et 71 microns
- 10 % de particules inférieures à 71 microns.

On fabrique à l'aide de cette poudre un chewing-gum de la formule suivante :

- gomme base 34/42          25 % en poids
- sirop de glucose hydrogéné de marque LYCASIN 80/55          20 % en poids
- maltitol poudre conforme aux analyses précédentes          55 % en poids
- arôme          1,2 % en poids
- colorant          quantité suffisante

Pour la fabrication, on ramollit la gomme base en la portant à une température d environ 70-80 °C et on la place dans un pétrin préchauffé à 45-50 °C.

On y ajoute le sirop de glucose hydrogéné de marque LYCASIN 80/55 préchauffé à 45° C et on pétrit pendant deux minutes.

On ajoute alors le tiers de la poudre de maltitol obtenue comme décrit précédemment et on pétrit encore durant deux minutes à la suite de quoi on introduit un nouveau tiers de la poudre de maltitol.

Après deux minutes de pétrissage, on ajoute le reste de la poudre de maltitol, puis l'arôme et le colorant.

On pétrit encore deux minutes, puis on lamine et découpe la pâte.

Soumis à un panel de dégustateurs spécialisés, le produit a été jugé supérieur aux chewing-gums "sans sucre" habituellement trouvés sur le marché. Le chewing-gum ainsi obtenu est tendre, élastique, non collant. La saveur sucrée est très agréable.

**Revendications**

1. Procédé pour la préparation simultanée d'un sirop riche en maltitol propre à l'obtention de maltitol cristallisé et d'un sirop riche en maltotriitol comprenant

   - la liquéfaction d'un lait d'amidon,
   - le traitement du lait d'amidon liquéfié par l'action d'une enzyme saccharifiante en vue de l'obtention d'un sirop de maltose,

- l'hydrogénation catalytique dudit sirop de maltose à l'aide de catalyseurs à base de Ruthenium ou de nickel Raney en vue de l'obtention d'un sirop de maltitol.

ledit procédé étant caractérisé par le fait

- que l'on soumet un lait d'amidon ayant une teneur en matières sèches de 25 à 45% en poids à une liquéfaction jusqu'à une valeur de dextrose-équivalent égale ou supérieure à 2,
- que l'on soumet le lait d'amidon liquéfié à une saccharification enzymatique jusqu'à obtention d'un sirop de maltose ayant une teneur en maltose de seulement 50 à 80% en poids,
- que l'on hydrogène ledit sirop de maltose par voie catalytique en vue de l'obtention d'un sirop de maltitol, ce sirop contenant également du sorbitol, du maltotriitol et des polyols de degré de polymérisation $\geqq$ 4.
- que l'on soumet ledit sirop de maltitol à un fractionnement chromatographique sur des résines cationiques fortement acides, chargées en ions alcalins ou alcalino-terreux, fournissant une fraction riche en sorbitol, une fraction riche en polyols de degré de polymérisation $\geqq$ 4, une fraction de sirop riche en maltotriitol et une fraction de sirop riche en maltitol, les conditions opératoires de ladite étape de fractionnement chromatographique ayant été choisies de telle sorte que ladite fraction de sirop riche en maltitol comprend, les pourcentages étant exprimés en poids sur la base de la teneur en matières sèches:

au moins 87% de maltitol, de préférence de 87 à 97,5% et, encore plus préférentiellement. de 87 à 95.5%, moins de 1% de polyols de degré de polymérisation $\geqq$ 4, de préférence moins de 0,70% et, plus préférentiellement encore, moins de 0,60%.
moins de 5% de sorbitol, de préférence moins de 3% et, encore plus préférentiellement, moins de 2%, une proportion de maltotriitol comprise entre 2,5 et 13% et,

- que l'on récupère ladite fraction de sirop riche en maltitol.

**Patentansprüche**

1. Verfahren zur gleichzeitigen Herstellung eines an Maltit reichen Sirups für die Herstellung von kristallisiertem Maltit und eines an Maltotriit reichen Sirups, umfassend

- die Verflüssigung einer Stärkemilch,
- die Behandlung der verflüssigten Stärkemilch mit einem saccharifizierenden Enzym zur Erzielung eines Maltosesirups,
- die katalytische Hydrierung des Maltosesirups mit Katalysatoren auf Basis von Ruthenium oder Raney-Nickel zur Erzielung eines Maltitsirups,

wobei das Verfahren dadurch gekennzeichnet ist, daß

- man eine Stärkemilch mit einem Trockensubstanzgehalt von 25 bis 45 Gew.-% einer Verflüssigung bis zu einem Dextroseäquivalent-Wert gleich oder höher als 2 unterzieht,
- man die verflüssigte Stärkemilch einer enzymatischen Saccharifizierung bis zur Erzielung eines Maltosesirups mit einem Maltosegehalt von lediglich 50 bis 80 Gew.-% unterzieht,
- man den Maltosesirup katalytisch zur Erzielung eines Maltitsirups hydriert, wobei dieser Sirup auch Sorbit, Maltotriit und Polyole mit einem Polymerisationsgrad $\geq$ 4 enthält,
- man den Maltitsirup einer chromatographischen Fraktionierung auf Stark gesäuerten, mit alkalischen oder erdalkalischen Ionen beladenen Kationenharzen unterzieht, die eine Sorbit-reiche Fraktion, eine an Polyolen mit einem Polymerisationsgrad $\geq$ 4 reiche Fraktion, eine Maltotriit-reiche Sirupfraktion und eine an Maltit reiche Sirupfraktion ergibt, wobei die Arbeitsbedingungen der chromatographischen Fraktionierungsstufe derart gewählt werden, daß die an Maltit reiche Sirupfraktion die folgenden Prozentanteile, ausgedrückt in Gewicht auf Basis des Trockensubstanzgehaltes, enthält:

mindestens 87 % Maltit, vorzugsweise 87 bis 97,5 % und insbesondere 87 bis 95,5 %, weniger als 1 % Polyole mit einem Polymerisationsgrad $\geq$ 4, vorzugsweise weniger als 0,70 % und insbesondere weniger als 0,60 %, weniger als 5 % Sorbit, vorzugsweise weniger als 3 % und insbesondere weniger als 2 %, einen Anteil an Maltotriit zwischen 2,5 und 13 % und

- man die an Maltit reiche Sirupfraktion gewinnt.

## Claims

1. Process for the simultaneous preparation of a syrup rich in maltitol suitable to obtain crystallized maltitol and of a syrup rich in maltotriitol comprising :

   - the liquefaction of a starch milk,
   - the treatment of the liquefied starch milk by the action of a saccharifying enzyme in order to obtain a maltose syrup,
   - the catalytic hydrogenation of the said maltose syrup with Ruthenium or Raney nickel catalysts in order to obtain a maltitol syrup,

   the said process being characterized by the fact

   - that a starch milk having a dry matter content of 25 to 45 % by weight is subjected to a liquefaction until a Dextrose-Equivalent value equal or higher than 2,
   - that the liquefied starch milk is subjected to an enzymatic saccharification until obtention of a maltose syrup having a maltose content from only 50 to 80 % by weight,
   - that the said maltose syrup is hydrogenated by enzymatic route in order to obtain a maltitol syrup, this syrup containing also sorbitol, maltotriitol and polyols of degree of polymerization $\geq 4$,
   - that the said maltitol syrup is submitted to a chromatographic fractionation on highly acid cationic resins, charged with alkaline or alkaline-earth ions, providing a fraction rich in sorbitol, a fraction rich in polyols of degree of polymerization $\geq 4$, a fraction of syrup rich in maltotriitol and a fraction of syrup rich in maltitol, the process conditions of the said chromatographic fractionation step being selected in such a way that the said fraction of syrup rich in maltitol comprises, the percentages being expressed by weight on the basis of the dry matter content :

     at least 87 % of maltitol, preferably from 87 to 97.5 % and, still more preferably, from 87 to 95.5 %,
     less than 1 % of polyols of degree of polymerization $\geq 4$, preferably less than 0.70 % and, still more preferably, less than 0.60 %,
     less than 5 % of sorbitol, preferably less than 3 % and, still more preferably, less than 2 %,
     a proportion of maltotriitol comprised between 2.5 % and 13 % and

   - that the said fraction of syrup rich in maltitol is recovered.

# FIG.3.

ZONE Ⅲ      ZONE I    ZONE Ⅱ

FIG.4.

FIG.1.

FIG.2.